# EUROPEAN PATENT SPECIFICATION

(11) **EP 1 273 301 B1**
(45) Date of publication and mention of the grant of the patent: **06.09.2006**
(21) Application number: 02015073.6
(22) Date of filing: 05.07.2002
(51) Int. Cl.: A61K 47/00, A61K 36/00

(54) **Pharmaceutical preparations based on active ingredients susceptible to illicit administration**
Pharmazeutische Zubereitungen, Wirkstoffe enthaltend, die sich für unerlaubte Verabreichung anbieten
Préparations pharmaceutiques comprenant des principes actifs susceptibles d'administration illicite

(30) Priority: 06.07.2001 IT MI20011446
(43) Date of publication of application: 08.01.2003
(73) Proprietor: ALTERGON S.A., CH-6903 Lugano (CH)
(72) Inventor: Garavani, Alberto, 6900 Massagno (CH); Marchiorri, Maurizio, 22039 Valbrona (Prov. of Como) (IT); Di Martino, Alessandro, 20123 Milano (IT)
(74) Representative: Gervasi, Gemma

(56) References cited:
- INTERNET, [Online] XP002221355 Retrieved from the Internet: <URL:http://news.bbc.co.uk/1/hi/uk/50369.s tm> [retrieved on 2002-11-19]

## Description

### Field of the invention

The present invention relates to pharmaceutical preparations based on active ingredients susceptible to illicit administration.

### Prior art

A plurality of active ingredients susceptible to illicit administration is known in the pharmaceutical field, e.g. those acting on the central nervous system and as narcotics. In relation to their specific activity, said active ingredients are classified e.g. as sedative, hypnotic, antipsychotic, antidepressant, tranquillising, and antimuscarinic agents.

For the patients' convenience and simplicity of administration, many of said active ingredients are marketed, in the form of pharmaceutical compositions adapted for oral administration, like e.g. tablets or capsules, whenever absorbability from the gastrointestinal tract and the resultant bioavailability of the so-formulated active ingredient are satisfactory. In fact, the possibility of modulating the active ingredient's pharmacokinetics by the use of excipients, coatings, by selecting the formulation procedures, such as for example micro- and nanoencapsulation, and by identifying a suitable crystalline or amorphous form, makes it possible to obtain a solid form of administration whereby most of said active ingredients can be administered by the oral way.

Unfortunately, however, the use of most of the aforesaid active ingredients is not only therapeutic, but also abusive or illicit. Out of the illicit uses, a distinction may be made between the voluntary illicit self-administration, e.g. by drug addicts, and the illicit administration to other people.

The illicit administration to other people is practised by criminals who exploit the narcotic effect produced by the active ingredient on the victim to commit further crimes against them or to induce drug addiction. By way of example, according to a piece of news dated June 27, 1998 and recently available on the internet (http://www.corpus-delicti.com), burundanga, a very potent form of scopolamine, has a dubious success in the criminal racket. Used for decades in Columbia by the native population in tribal rituals, even very small doses of the drug are reported to cause "submissive" behaviour in a victim, while larger doses cause instantaneous unconsciousness, followed even by complete amnesia.

While the strong hypnotic and amnesic effects of scopolamine have been exploited for quite a time, e.g. in California, by criminals to drug and rob their victims, the recent decrease in crimes is mostly to be attributed to the lower availability of preparations containing scopolamine on the black market, as other less hazardous active ingredients have displaced it for legitimate medical uses. In any case, the problem of illicit administration has become increasingly serious, as proved by the fact that the illicit use of burundanga accounts for more than half of emergency room admissions and for over 500 reported crimes per month solely in Columbia.

According to recent news, there has been increasing abuse-though to a lesser extent-of another active ingredient, i.e. flunitrazepam.

Although some pharmaceutical preparations produced and marketed by the industry necessitate medical prescription and medical supervision during administration, they are often available through criminal routes and, further to the natural extracts of dubious origin, they constitute a major source of active ingredients acting on the central nervous system and as narcotics for illicit use.

In practice, while all types of commercial-scale pharmaceutical preparations are suitable for voluntary abusive self-administration, their illicit administration to other people is mostly limited to "hidden" administration, i.e. without the knowledge of the victim. In particular, drugs for illicit administration are mostly in the oral, often solid, form and are introduced, often in high doses, into drinks or foodstuff.

Illicit administration seems to be a problem also in the world of professional sport. In particular, after the introduction of increasingly sensitive and stringent anti-doping controls, an increased number of sportsmen provide evidence of their being treated-without their knowing it-with anabolic agents or the like, suitable for enhancing their sportive performance and/or increasing the size of their muscles.

Even if the abusive or illicit administration of commercial drugs is beyond the pharmaceutical industry's control, any abuse of this type is clearly a serious ethical problem for manufacturers. Therefore, the need for oral pharmaceutical preparations unsuitable for illicit administration is deeply felt.

### Summary

The above object and other objects -which will better appear from the following description-are met by a pharmaceutical formulation to be taken orally in the form of a soft capsule according to claim 1 enclosing an active ingredient susceptible to illicit administration and a pharmaceutically acceptable organoleptic marker, particularly evident for its odour, taste or colour or for its scarce miscibility with food.

### Detailed description of the invention

The illicit administration of active ingredients to other people is a serious crime.

Unfortunately, there are many active ingredients susceptible to illicit administration; in fact, the effects they produce on the victims bring about criminal advantages to the suppliers. By way of example, mention is made of the substances acting on the central nervous system and/or as narcotics, the anabolic agents and the like. In any case, the present invention relates to all active ingredients susceptible to illicit administration.

Illicit administration of substances acting on the nervous system and/or as narcotics is practised by criminals to "drug" or even poison the victims and thus cause their losing consciousness, as well as by drug dealers to induce addiction in perspective "clients". By way of example, in the case of crimes of this type committed between relatives, the criminal subject sometimes enjoys the victim's confidence.

As concerns the illicit administration of anabolic agents or the like, criminal and victim are always bound by a well-established relation of trust, which may be viciously exploited by the criminal to manipulate food.

In both aforementioned cases, the pharmaceutical forms for oral administration obtained illicitly are mixed with food or drinks. In particular, the solid forms, such as tablets, are ground and dissolved in drinks or mixed with food, while the capsules (enclosing a liquid or a solid) are emptied and their contents are mixed with food or drinks. After said treatment, the drug added to food or drinks even in substantial amounts cannot be perceived by the victim. This is particularly true when the drug is mixed e.g. with a drink with a strong or anyhow unusual taste (e.g. an alcoholic cocktail given to a young victim), and when it is drunk in a dimly lit place, e.g. a discotheque. Consequently, the drug is taken up by the victim which remains unaware and does not notice anything suspicious with the such manipulated food.

To prevent such food manipulation, the present invention provides a pharmaceutical product for oral administration that contains, in addition to the active ingredient susceptible to illicit administration, one or more substances having evident organoleptic properties in aqueous or oily or alcoholic suspension/solution. Said substances, the so-called organoleptic markers, which make the illicit administration to victims impossible, must be perfectly edible and harmless in the proposed concentrations, and must not be perceived upon correct use of the drug. In particular, the organoleptic markers, besides being pharmaceutically acceptable, must be so incorporated into the oral pharmaceutical preparation that they become perceptible only upon illicit mixing with food and drinks.

Out of the active ingredients susceptible to illicit administration, those acting on the nervous system and as narcotics undoubtedly account for most abuses, whereas anabolic agents or the like seem to constitute a small-scale, but growing "market".

With regard to the active ingredients acting on the nervous systems and as narcotics, the present invention refers to all substances of the type susceptible to illicit use, if any. In particular, the present invention relates to sedative, hypnotic, antipsychotic, antidepressant, tranquillising, and antimuscarinic substances, in particular all substances depressing the central nervous system. By way of example, not of limitation, the present invention concerns the following openended listing of active ingredients and combinations thereof: abecarnil, acamprosate calcium, acepromazine (+maleate), aceprometazine, acetophenazine maleate, acetylglycinamide-chloralum, adinazolam mesylate, allobarbitone, aldipem, alprazolam, amisulpride, amylobarbitone (+sodium), aprobarbital, azaperone, barbitone (+sodium), benperidol, bentazepam, brallobarbital, bromazepam, bromisoval, bromperidol (+decanoate), brotizolam, buspirone hydrochloride, butalbital, butobarbitone, calcium bromolactobionate, camazepam, captodiame hydrochloride, carbromal, carpipramine hydrochloride, chloral betaine, chloralum, chloralose, chlordiazepoxide (+hydrochloride), chlorhexadol, chlormethiazole (+edisylate), chlormezanone, chloproethazine hydrochloride, chlorpromazine (+embonate and hydrochloride), chlorprotixene (+hydrochloride and mesylate), cinolazepam, clobazam, clocapramine hydrochloride, chlorazepine acid, (+potassium salts), clotiapina, clothiazepam, cloxazolam, clozapine, cyamemazine, cyclobarbitone (+calcium), delorazepam, detomidin hydrochloride, desmedetomidine, diazepam, dichloralphenazone, difebarbamate, droperidole, eltoprazine, enciprazine hydrochloride, estazolam, etclorvinol, ethyl loflazepate, etifoxine hydrochloride, etizolam, etodroxizine, febarbamate, fluanisone, fludiazepam, flunitrazepam, flupentixol decanoate (+hydrochloride), fluphenazine decanoate (+enanthate and hydrochloride), flurazepam, (+monohydrochloride and dihydrochloride), fluspirilene, gepirone hydrochloride, glutethimide, halazepam, haloperidol, haloxazolam, hexapropymate, hexobarbitone (+sodium), homofenazine hydrochloride, ibomal, ipsapirone hydrochloride, ketazolam, loprazolam mesylate, lorazepam, lormetazepam, loxapine, magnesium aspartate hydrobromide, medazepam, medetomidine hydrochloride, melperone hydrochloride, mephenoxalone, meprobamate, mesoridazine besylate, metaclazepam hydrochloride, methaqualone, methotrimeprazine, methylpentynol, mexazolam, midazolam (+maleate), molindone hydrochloride, moperone hydrochloride, mosapramine, nemonapride, nimetazepam, nitrazepam, nordazepam, olanzapine, oxazepam, ossazolam, oxypertine, paraldehyde, penfluridol, pentobarbitone (+calcium and sodium), perazine dimalonate, periciazine, perphenazine (+decanoate and enanthate), phenprobamate, pimozide, pinazepam, pipamperone hydrochloride, pipothiazine, prazepam, prochlorperazine (+edisylate, maleate and mesylate), prolonium bromide, promazine embonate, prothipendyl hydrochloride, proxibarbal, pyrithyldione, quazepam, quetiapine fumarate, quinalbarbitone (+sodium), raclopride, remoxipiride hydrochloride, risperidone, ritanserine, romifidine, secbutobarbitone (+sodium), sertindole, sulpiride, sultopride hydrochloride, suriclone, tandospirone citrate, temazepam, tetrabenazine, tetrazepam, thioproperazine mesylate, thioridazine, thiothixene (+hydrochloride), thiapride hydrochloride, timiperone, tofisopam, triazolam, triclofos sodium, trifluoperazine hydrochloride, trifluperidol (+hydrochloride), valnoctamide, veralipride, vinylbitone, zaleplon, ziprasidone, zolazepam hydrochloride, zolpidem tartrate, zopiclone, zotepine, zuclopenthixol (+acetate, decanoate, hydrochloride), atropine (+methobromide, methonitrate and sulphate), belladonna, benzhexol hydrochloride, benztropine mesylate, biperiden, bomaprine hydrochloride, butropium bromide, buzepide metiodure, cimetropium bromide, clidinium bromide, ciclodrine hydrochloride, cyclopentolate hydrochloride, darifenacin, desetimide hydrochloride, diclomine hydrochloride, diethazine hydrochloride, difemerine hydrochloride, dihexiverine hydrochloride, dimevamide, diphemanil methyl sulphate, drofenine hydrochloride, emepronium bromide (+carragenate), ethopropazine hydrochloride, eucatropine hydrochloride, fentonium bromide, flavoxate hydrochloride, flutropium bromide, glycopyrronium bromide, homatropine (+hydrobromide and methobromide), hioscine (+butylbromide, hydrobromide, methobromide and methonitrate), hiosciamine, hyosciamus, isopropamide iodide, mecloxamine citrate, mepenzolate bromide, methanthelinium bromide, metixene hydrochloride, metilbenactizium bromide, octatropine methylbromide, orphenadrine citrate (+hydrochloride), oxybutinin hydrochloride, oxyphencyclimine hydrochloride, oxyphenonium bromide, penthienate bromide, fenamazide hydrochloride, pipenzolate bromide, piperidolate hydrochloride, pipethanate ethobromide, pirenzepine hydrochloride, poldine methyl sulphate, pirifinium bromide, procyclidine hydrochloride, propanteline bromide, propiverine hydrochloride, stramonium, telenzepine, terodiline hydrochloride, tiemonium iodide, timepidium bromide, tolterodine tartrate, tridihexethyl chloride, tropatepine hydrochloride, tropicamide, tropine benzylate hydrochloride, valethamate bromide, xenytropium bromide, zamifenacin, amesergide, amineptine hydrochloride, amitriptiline, amoxapine, befloxatone, benactizine hydrochloride, brofaromine, buproprion hydrochloride, butriptylin hydrochloride, citalopram hydrobromide, clomipramine hydrochloride, clorgiline hydrochloride, demexiptiline hydrochloride, desipramine hydrochloride, dibenzepine hydrochloride, dothiepin hydrochloride, doxepin hydrochloride, duloxetine hydrochloride, etoperidone hydrochloride, femoxetine, fluoxetine hydrochloride, fluvoxamine maleate, imipramine, iprindole hydrochloride, iproniazid phosphate, isocarboxazid, lithium carbonate (+citrate), lofepramine hydrochloride, maprotiline hydrochloride, medifoxamine, melitracen hydrochloride, metapramine fumarate, mianserine hydrochloride, milnacipran, minaprine hydrochloride, mirtazapine, moclobemide, nefazodone hydrochloride, nialamide, nomifenisine maleate, nortriptyline hydrochloride, opipramol hydrochloride, oxaflozan hydrochloride, oxaprotiline hydrochloride, oxytriptan, paroxetine hydrochloride, phenelzine sulphate, pirlindole, pivagabine, protriptiline hydrochloride, quinupramine, reboxetine, rubidium chloride, sertraline hydrochloride, tianeptine sodium, tranylcypromine sulphate, trazodone hydrochloride, trimipramine (+maleate), tryptophan, venlafaxine hydrochloride, viloxazine hydrochloride, viqualine, zimeldine hydrochloride, alphadolone acetate, alphaxalone, eltanolone, etomidate, ketamine hydrochloride, methohexitone, propanidid, propofol, sodium oxybate, thiamilal sodium, tiopenthone sodium, tiletamine hydrochloride and others, identified e.g. in Martindale's Complete Drug Reference, Pharmaceutical Press, 1999.

As concerns active ingredients with anabolising activity or the like, the present invention relates to all substances susceptible to illicit use, if any. In particular, the present invention contemplates the substances suitable for enhancing the sportsmen's performance and/or increasing the size of their muscles, e.g. anabolising steroids. By way of example, not of limitation, the present invention concerns the following active ingredients and combinations thereof: testosterone, clostebole acetate, drostanolone propionate, estrapronicate, ethylestrenol, fluoximesterone, formebolone, mepitiostan, mesterolone, metandienone, metenolone acetate, methyltestosterone, nandrolone (+cyclohexylpropionate, decanoate, laurate, phenylpropionate, sodium sulphate and undecanoate), norethandrolone, oxabolone cipionate, oxandrolone, oxymetholone, stanozolol and trenbolone acetate and others, identified e.g. in Martindale's Complete Drug Reference, Pharmaceutical Press, 1999.

As concerns organoleptic markers, the present invention contemplates the use of one or more substances that, not withstanding their pharmaceutical acceptability , are particularly evident because of their odour or taste or colour or for their scarce miscibility with food. The preferred organoleptic markers according to the present invention are selected from the groups consisting of pharmaceutically acceptable hydrophilic flavouring agents, hydrophobic flavouring agents, hydrophilic colouring agents, hydrophobic colouring agents, odorants, flavours and scents (hydrophobic and hydrophilic) and oils. By way of example, not of limitation, the present invention concerns the following organoleptic markers and combinations thereof:

| Garlic (Allium sativum [Liliaceae] | As extract or one or more of its constituents: |
|---|---|
| | - Alliine |
| | - Allicine |
| | - Diallylsulphide |
| Aloe vera extract (Aloe barbadensis = Aloe vera. Aloe capensis, Aloe ferox [Liliaceae]) Quinine sulphate/hydrochloride/bromohydrate | - Ajoene |
| (Cinchona [Rublaceae]) | As extract or one or more of its components |
| | or quina extract as is |
| Chili Pepper | pure, as extract or components thereof, in particular capsicine |
| Natural colouring agents: | E100, E101, E140, E160, E160a, E160b, E160c, E160d, E160e, E160f, E161, E161a, E161b, E161c, E161d, E161e, E161f, E161g, E162, E163, saffron |
| Sundry colouring agents: | E104, E110. E120, E122, E123, E124, E127, E131, E132, E141, E142, E150, E151, E153 |

Examples of pharmaceutically acceptable oils are soybean oil, refined palm oil, hydrogenated coconut oil, castor oil and cod-liver oil; whereas further organoleptic markers scarcely miscible with food and drinks include pharmaceutically acceptable fats and waxes.

Particularly preferred are all organoleptic markers perceived as "strange" by the victim as they have unusual odours/colours/flavours combinations, such as for example garlic/yellow/bitter or cheese/violet/pungent. As concerns the pharmaceutical formulations for oral administration, the present invention contemplates soft capsules. In particular, the present Within the scope of the present invention, the phrase "soft capsule" designates-indipendently from the amount of plasticizer present in the shell--all airtight capsules, i.e. suitable for enclosing liquid phases, even if not microencapsulated.

According to the embodiments of the present invention, one or more of the aforesaid organoleptic markers are incorporated into soft capsules. In particular, the markers used are e.g. hydrophilic flavouring agents with a particularly bitter or unpleasant or pungent taste, preferably combined with hydrophilic colouring agents with a particularly strong and brilliant colour, unusual for foodstuffs, e.g. green, yellow, red, violet.

A further organoleptic marker that may be incorporated into the pharmaceutical preparations according to the present invention consists of unpleasant and disgusting odorants and scents, capable of vexing the smell. The odorants producing an unpleasant odour especially in the presence of alcohol are particularly preferred.

An organoleptic marker that is incorporated into the pharmaceutical preparations according to the present consists of substances that are scarcely miscible with foodstuffs, in particular immiscible with non-strongly alcoholic drinks. Said organoleptic markers hinder the mixing of drugs with foodstuffs since they bring about the formation of biphase systems easily perceived by the victim. An example of an organoleptic marker that may be hardly mixed with foodstuff may consists of a fat, in particular of a lipidic substance.

A soft capsule is thus obtained which -upon grinding and especially upon mixing with food or drinks-by releasing of the organoleptic markers present therein produces a mixture which is practically inedible because of its taste or unpleasant or disgusting odour; or that, in any case-indicates to the victim, due to its taste or odour or to its unusual colour or appearance- that the mixture has been manipulated. Especially when the manipulation of food or drinks with the pharmaceutical formulations of the invention results in the production of an unpleasant odour, the criminal may even desist from offering said mixture to the victim. Furthermore, unpleasant odour and/or taste may, within certain limits, act as a deterrent to abusive self-administration, especially when-as is often the case--a drug addict or a perspective suicide hopes to facilitate or speed up self-administration of a drug in very high doses, by using a drink as a vehicle.

According to the preferred embodiments of the invention one or more organoleptic markers as described above are enclosed in a soft capsule together with the active ingredient susceptible to illicit administration and the further organoleptic marker, particularly evident for its scarce miscibility with foodstuff. This last marker consists of a pharmaceutically acceptable and edible oil, such as for example castor oil or cod-liver oil. In fact, due to the presence of an oily fraction, said pharmaceutical formulations, to be administered by the oral way, require the use of a soft capsule. In practice, the oily fraction, which may form stains on the surface of a non-strongly alcoholic drink, may act as a floating "buoy" warning the perspective victim against illicit manipulation.

Importantly, said preferred embodiments of the invention can be advantageously applied not only when the form of administration according to the prior art already consists of a hard or soft capsule, but also whenever the conventional form of administration consists of a tablet or pill or syrup, etc.

In particular, the soft capsules according to the invention, which enclose an active ingredient susceptible to illicit administration and an organoleptic marker immiscible with non-strongly alcoholic drinks, may also contain a combination of other hydrophilic and hydrophobic organoleptic markers.

According to a first particularly preferred embodiment of the present invention, soft capsules are provided that enclose an oily suspension comprising a liposoluble active ingredient dissolved in the oil, at least a first liposoluble organoleptic marker and at least a second water soluble organoleptic marker suspended in it.

Therefore, in the case of liposoluble (or in any case slightly water soluble) active ingredients susceptible to illicit administration, such as for example chlorpromazine, etclorvinol, flunitrazepam or some anabolising steroids, the soft capsule according to a particularly preferred embodiment of the present invention encloses an oil wherein the active ingredient, a first colouring agent and preferably a first hydrophobic flavouring agent have been dissolved. Furthermore, a second hydrophilic colouring agent with very high staining power, e.g. saffron, and preferably a second hydrophilic odorant and/or flavouring agent have been suspended in the oil enclosed in the soft capsule.

Consequently, the lipophilic active ingredient is entirely protected from illicit administration because:
- the soft capsule contents, once drawn by a syringe, are immiscible with drinks, with the exception of strong alcoholic drinks;
- in particular, when the pharmaceutical capsule oily contents come into contact with a non-strongly alcoholic drink, a coloured oil stain with a disgusting taste (originated from the first hydrophobic organoleptic markers) forms on the drink surface;
- furthermore, when trying to mix the resulting biphase system, the oily phase releases the second hydrophilic colouring agent (e.g. saffron) and optionally the second hydrophilic odorant and flavouring agent to the drink. As a result of the attempted illicit manipulation, the drink becomes undrinkable, even after removal of the unpleasant oily phase (and of the active ingredient);
- conversely, in the case of strong alcoholic drinks, notwithstanding the probable active ingredient extraction from the oily phase and the at least partial solubility of the oily phase in strong drinks, the hydrophilic and hydrophobic organoleptic markers, used together, make the manipulated strong alcoholic drink taste unpleasant, even without formation of the biphase system;
- furthermore, by way of the organoleptic markers contents of hydrophilic and hydrophobic nature, also the mixing of the oily phase enclosed in the capsule with solid foodstuff spoils taste of the food irreversibly.

According to another particularly preferred embodiment of the present invention, soft capsules are provided that enclose an oily suspension including a water soluble active ingredient suspended in the oil, at least a first water soluble organoleptic marker, and at least a second liposoluble organoleptic marker dissolved in the oil.

In fact, the formulations in the form of soft capsules enclosing organoleptic markers, scarcely miscible with foodstuff, are equally suitable for water soluble (or prevailingly water soluble) active ingredients susceptible to illicit administration, such as for example barbiturates, scopolamine, hioscine or various addition salts or active ingredient prodrugs. In this case, the soft capsule according to the present preferred embodiment of the invention encloses an oil wherein the hydrophilic active ingredient and a first hydrophilic colouring agent with very high staining power, e.g. saffron, and preferably a first hydrophilic odorant and/or flavouring agent have been suspended. The oil includes a second hydrophobic colouring agent and preferably a second hydrophobic flavouring agent, both being dissolved therein.

As in the case described above and despite the rather low extraction--upon illicit mixing--of the active ingredient from the oily suspension by any drink (indipendently from its alcohol content), the final drink--also after removal of the exhausted oily phase--is always perceptibly modified by at least some organoleptic markers (in particular, the hydrophilic ones and, in the case of strong alcoholic drinks, also the lipophilic ones) contained in the particularly preferred pharmaceutical composition of the invention. This obviously holds also for the mixture with solid food.

### Experimental part

### Examples:

The following tables, given by way of illustration, show some liquid or semi-liquid formulations, used in soft capsules according to the present invention:

### Contents of soft capsules enclosing oily organoleptic markers:

| Example 1 | Example 2 |
|---|---|
| Flunitrazepam | Scopolamine (or hyoscine) |
| Soybean oil | Soybean oil |
| Refined palm oil | Refined palm oil |
| Hydrogenated coconut | Hydrogenated coconut |
| oil | oil |
| Yellow bees-wax | Yellow bees-wax |
| Garlic oil (liposoluble) | Garlic oil (liposoluble) |
| Saffron (water soluble) | Saffron (water soluble) |
| Lecithin | Lecithin |
| Gelatin | Gelatin |
| Glycerol | Glycerol |

## Claims

1. Pharmaceutical formulation for oral administration in the form of a soft capsule enclosing a water soluble or liposoluble active principle susceptible to illicit administration and at least one pharmaceutically acceptable organoleptic marker independently selected out of one or more substances belonging to the group consisting of a hydrophilic flavouring agent, a hydrophobic flavouring agent, a hydrophilic colouring agent, a hydrophilic colouring agent, a hydrophobic colouring agent an an odorant, wherein the said pharmaceutical formulation contains an oily suspension or solution comprising the active principle dissolved or suspended in the oil, at least a first water soluble organoleptic marker suspended in the oil, at least a second liposoluble organoleptic marker dissolved in the oil.

2. The pharmaceutical formulation as claimed in claim 1 wherein the active principle is selected from the group consisting of a substance acting on the central nervous system and/or as a narcotic and of a substance with anabolising activity or the like.

3. The pharmaceutical formulation as claimed in claim 2 wherein the active principle acting on the central nervous system and/or as a narcotic is selected from the group consisting of sedative, hypnotic, antipsychotic, antidepressant, tranquillising, and antimuscarinic agents.

4. The pharmaceutical formulation as claimed in claim 3 wherein the active ingredient acting on the central nervous system and/or as a narcotic is selected from the group consisting of abecarnil, acamprosate calcium, acepromazine (+maleate), aceprometazine, acetophenazine maleate, acetylglycinamide-chloralum, adinazolam mesylate, allobarbitone, aldipem, alprazolam, amisulpride, amylobarbitone (+sodium), aprobarbital, azaperone, barbitone (+sodium), benperidol, bentazepam, brallobarbital, bromazepam, bromisoval, bromperidol (+decanoate), brotizolam, buspirone hydrochloride, butalbital, butobarbitone, calcium bromolactobionate, camazepam, captodiame hydrochloride, carbromal, carpipramine hydrochloride, chloral betaine, chloralum, chloralose, chlordiazepoxide (+hydrochloride), chlorhexadol, chlormethiazole (+edisylate), chlormezanone, chloproethazine hydrochloride, chlorpromazine (+embonate and hydrochloride), chlorprotixene (+hydrochloride and mesylate), cinolazepam, clobazam, clocapramine hydrochloride, chlorazepine acid, (+potassium salts), clotiapina, clothiazepam, cloxazolam, clozapine, cyamemazine, cyclobarbitone (+calcium), delorazepam, detomidin hydrochloride, desmedetomidine, diazepam, dichloralphenazone, difebarbamate, droperidole, eltoprazine, enciprazine hydrochloride, estazolam, etclorvinol, ethyl loflazepate, etifoxine hydrochloride, etizolam, etodroxizine, febarbamate, fluanisone, fludiazepam, flunitrazepam, flupentixol decanoate (+hydrochloride), fluphenazine decanoate (+enanthate and hydrochloride), flurazepam, (+monohydrochloride and dihydrochloride), fluspirilene, gepirone hydrochloride, glutethimide, halazepam, haloperidol, haloxazolam, hexapropymate, hexobarbitone (+sodium), homofenazine hydrochloride, ibomal, ipsapirone hydrochloride, ketazolam, loprazolam mesylate, lorazepam, lormetazepam, loxapine, magnesium aspartate hydrobromide, medazepam, medetomidine hydrochloride, melperone hydrochloride, mephenoxalone, meprobamate, mesoridazine besylate, metaclazepam hydrochloride, methaqualone, methotrimeprazine, methylpentynol, mexazolam, midazolam (+maleate), molindone hydrochloride, moperone hydrochloride, mosapramine, nemonapride, nimetazepam, nitrazepam, nordazepam, olanzapine, oxazepam, ossazolam, oxypertine, paraldehyde, penfluridol, pentobarbitone (+calcium and sodium), perazine dimalonate, periciazine, perphenazine (+decanoate and enanthate), phenprobamate, pimozide, pinazepam, pipamperone hydrochloride, pipothiazine, prazepam, prochlorperazine (+edisylate, maleate and mesylate), prolonium bromide, promazine embonate, prothipendyl hydrochloride, proxibarbal, pyrithyldione, quazepam, quetiapine fumarate, quinalbarbitone (+sodium), raclopride, remoxipiride hydrochloride, risperidone, ritanserine, romifidine, secbutobarbitone (+sodium), sertindole, sulpiride, sultopride hydrochloride, suriclone, tandospirone citrate, temazepam, tetrabenazine, tetrazepam, thioproperazine mesylate, thioridazine, thiothixene (+hydrochloride), thiapride hydrochloride, timiperone, tofisopam, triazolam, triclofos sodium, trifluoperazine hydrochloride, trifluperidol (+hydrochloride), valnoctamide, veralipride, vinylbitone, zaleplon, ziprasidone, zolazepam hydrochloride, zolpidem tartrate, zopiclone, zotepine, zuclopenthixol (+acetate, decanoate, hydrochloride), atropine (+methobromide, methonitrate and sulphate), belladonna, benzhexol hydrochloride, benztropine mesylate, biperiden, bornaprine hydrochloride, butropium bromide, buzepide metiodure, cimetropium bromide, clidinium bromide, ciclodrine hydrochloride, cyclopentolate hydrochloride, darifenacin, desetimide hydrochloride, diclomine hydrochloride, diethazine hydrochloride, difemerine hydrochloride, dihexiverine hydrochloride, dimevamide, diphemanil methyl sulphate, drofenine hydrochloride, emepronium bromide (+carragenate), ethopropazine hydrochloride, eucatropine hydrochloride, fentonium bromide, flavoxate hydrochloride, flutropium bromide, glycopyrronium bromide, homatropine (+hydrobromide and methobromide), hioscine (+butylbromide, hydrobromide, methobromide and methonitrate), hiosciamine, hyosciamus, isopropamide iodide, mecloxamine citrate, mepenzolate bromide, methanthelinium bromide, metixene hydrochloride, metilbenactizium bromide, octatropine methylbromide, orphenadrine citrate (+hydrochloride), oxybutinin hydrochloride, oxyphencyclimine hydrochloride, oxyphenonium bromide, penthienate bromide, fenamazide hydrochloride, pipenzolate bromide, piperidolate hydrochloride, pipethanate ethobromide, pirenzepine hydrochloride, poldine methyl sulphate, pirifinium bromide, procyclidine hydrochloride, propanteline bromide, propiverine hydrochloride, stramonium, telenzepine, terodiline hydrochloride, tiemonium iodide, timepidium bromide, tolterodine tartrate, tridihexethyl chloride, tropatepine hydrochloride, tropicamide, tropine benzylate hydrochloride, valethamate bromide, xenytropium bromide, zamifenacin, amesergide, amineptine hydrochloride, amitriptiline, amoxapine, befloxatone, benactizine hydrochloride, brofaromine, buproprion hydrochloride, butriptylin hydrochloride, citalopram hydrobromide, clomipramine hydrochloride, clorgiline hydrochloride, demexiptiline hydrochloride, desipramine hydrochloride, dibenzepine hydrochloride, dothiepin hydrochloride, doxepin hydrochloride, duloxetine hydrochloride, etoperidone hydrochloride, femoxetine, fluoxetine hydrochloride, fluvoxamine maleate, imipramine, iprindole hydrochloride, iproniazid phosphate, isocarboxazid, lithium carbonate (+citrate), lofepramine hydrochloride, maprotiline hydrochloride, medifoxamine, melitracen hydrochloride, metapramine fumarate, mianserine hydrochloride, milnacipran, minaprine hydrochloride, mirtazapine, moclobemide, nefazodone hydrochloride, nialamide, nomifenisine maleate, nortriptyline hydrochloride, opipramol hydrochloride, oxaflozan hydrochloride, oxaprotiline hydrochloride, oxytriptan, paroxetine hydrochloride, phenelzine sulphate, pirlindole, pivagabine, protriptiline hydrochloride, quinupramine, reboxetine, rubidium chloride, sertraline hydrochloride, tianeptine sodium, tranylcypromine sulphate, trazodone hydrochloride, trimipramine (+maleate), tryptophan, venlafaxine hydrochloride, viloxazine hydrochloride, viqualine, zimeldine hydrochloride, alphadolone acetate, alphaxalone, eltanolone, etomidate, ketamine hydrochloride, methohexitone, propanidid, propofol, sodium oxybate, thiamilal sodium, tiopenthone sodium, tiletamine hydrochloride.

5. The pharmaceutical formulation as claimed in claim 2 wherein the water soluble active principle with anabolising activity or the like is selected from the group consisting of substances suitable for enhancing the sportsmen's performance and/or increasing the muscular mass.

6. The pharmaceutical formulation as claimed in claim 5 wherein the active principle with anabolising activity or the like is selected from the group consisting of testosterone, clostebole acetate, drostanolone propionate, estrapronicate, ethylestrenol, fluoximesterone, formebolone, mepitiostan, mesterolone, metandienone, metenolone acetate, methyltestosterone, nandrolone (+cyclohexylpropionate, decanoate, laurate, phenylpropionate, sodium sulphate and undecanoate), norethandrolone, oxabolone cipionate, oxandrolone, oxymetholone, stanozolol and trenbolone acetate.

7. The pharmaceutical formulation as claimed in one of the preceding claims wherein the organoleptic markers are selected from the group consisting of garlic extract (allium sativum [Liliaceae]) or constituents thereof, e.g. alliine, allicine, diallylsulphide or ajoene, of aloe vera extract (aloe barbadensis, aloe vera, aloe capensis, aloe ferox [Liliaceae]) or one or more components thereof, of quinine sulphate/hydrochloride/bromohydrate (Cinchona [Rubiaceae]), or quina extract as is, of natural colouring agents, e.g. E100, E101, E140, E160a, E160b, E160c, E160d; E160e; E160f, E161, E161a, E161b, E161c, E161d, E161e, E161f, E161g, E162, E163, saffron, of sundry colouring agents, e.g. E104, E110, E120, E122, E123, E124, E127, E131, E132, E141, E142, E150, E151 and E153.

## Patentansprüche

1. Pharmazeutische Formulierung für die orale Verabreichung in Form einer weichen Kapsel beinhaltend einen wasser- oder fettlöslichen Wirkstoff, der sich für unerlaubte Verabreichung anbietet und mindestens einen pharmazeutisch akzeptablen organoleptischen Marker, welcher unabhängig unter einer oder mehreren Substanzen ausgewählt wird, welche zur Gruppe bestehend aus einem hydrophilen Geschmacksstoff, einem hydrophoben Geschmacksstoff, einem hydrophilen Farbstoff, einem hydrophoben Farbstoff und einem Geruchsstoff gehören, wobei die genannte pharmazeutische Formulierung eine ölige Suspension oder eine Lösung enthält, die den im Öl gelösten oder suspendierten Wirkstoff, mindestens einen ersten wasserlöslichen im Öl suspendierten organoleptischen Marker und mindestens einen zweiten fettlöslichen im Öl gelösten organoleptischen Marker umfaßt.

2. Pharmazeutische Formulierung gemäß Anspruch 1, wobei der Wirkstoff ausgewählt wird aus der Gruppe bestehend aus einer auf das Zentralnervensystem wirkenden Substanz und/oder einem Narkotikum und einer Substanz mit anabolischer oder ähnlicher Wirkung.

3. Pharmazeutische Formulierung gemäß Anspruch 2, wobei der Wirkstoff, der auf das Zentralnervensystem und/oder als Narkotikum wirkt, ausgewählt wird aus der Gruppe bestehend aus Sedativa, Hypnotika, Antipsychotika, Antidepressiva, Beruhigungsmitteln und Antimuskarin-Agenzien.

4. Pharmazeutische Formulierung gemäß Anspruch 3, wobei der aktive Inhaltsstoff, der auf das Zentralnervensystem und/oder als Narkotikum wirkt, ausgewählt wird aus der Gruppe bestehend aus Abecarnil, Acamprosat-Kalzium, Acepromazin (+Maleat), Aceprometazin, Acetophenazinmaleat, Acetylglycinamid-chloralum, Adinazolammesylat, Allobarbital, Aldipem, Alprazolam, Amisulprid, Amylobarbital (+Natrium), Aprobarbital, Azaperon, Barbital (+Natrium), Benperidol, Bentazepam, Brallobarbital, Bromazepam, Bromisoval, Bromperidol (+Dekanoat), Brotizolam, Buspironhydrochlorid, Butalbital, Butobarbital, Kalziumbromolactobionat, Camazepam, Captodiamhydrochlorid, Carbromal, Carpipraminhydrochlorid, Chloralbetain, Chloralum, Chloralose, Chlordiazepoxid (+Hydrochlorid), Chlorhexadol, Chlormethiazol (+Edisylat), Chlormezanon, Chloproethazinhydrochlorid, Chlorpromazin (+Embonat und Hydrochlorid), Chlorprotixen (+Hydrochlorid und mesylat), Cinolazepam, Clobazam, Clocapraminhydrochlorid, Chlorazepinsäure, (+Kaliumsalze), Clotiapina, Clothiazepam, Cloxazolam, Clozapin, Cyamemazin, Cyclobarbital (+Kalzium), Delorazepam, Detomidinhydrochlorid, Desmedetomidin, Diazepam, Dichloralphenazon, Difebarbamat, Droperidol, Eltoprazin, Enciprazinhydrochlorid, Estazolam, Etclorvinol, Ethylloflazepat, Etifoxinhydrochlorid, Etizolam, Etodroxizin, Febarbamat, Fluanison, Fludiazepam, Flunitrazepam, Flupentixoldekanoat (+Hydrochlorid), Fluphenazindekanoat (+Enanthat und Hydrochlorid), Flurazepam, (+Monohydrochlorid und Dihydrochlorid), Fluspirilen, Gepironhydrochlorid, Glutethimid, Halazepam, Haloperidol, Haloxazolam, Hexapropymat, Hexobarbital (+Natrium), Homofenazin-5-Hydrochlorid, Ibomal, Ipsapironhydrochlorid, Ketazolam, loprazolammesylat, lorazepam, lormetazepam, Loxapin, Magnesiumaspartathydrobromid, Medazepam, Medetomidinhydrochlorid, Melperonhydrochlorid, Mephenoxalon, Meprobamat, Mesoridazinbesylat, Metaclazepamhydrochlorid, Methaqualon, Methotrimeprazin, Methylpentynol, Mexazolam, Midazolam (+Maleat), Molindonhydrochlorid, Moperonhydrochlorid, Mosapramin, Nemonaprid, Nimetazepam, Nitrazepam, Nordazepam, Olanzapin, Oxazepam, Ossazolam, Oxypertin, Paraldehyd, Penfluridol, Pentobarbital (+Kalzium und Natrium), Perazindimalonat, Periciazin, Perphenazin (+Dekanoat und Enanthat), Phenprobamat, Pimozid, Pinazepam, Pipamperonhydrochlorid, Pipothiazin, Prazepam, Prochlorperazin (+Edisylat, Maleat und Mesylat), Proloniumbromid, Promazinembonat, Prothipendylhydrochlorid, Proxibarbal, Pyrithyldion, Quazepam, Quetiapinfumarat, Quinalbarbal (+Natrium), Racloprid, Remoxipiridhydrochlorid, Risperidon, Ritanserin, Romifidin, Secbutobarbal (+Natrium), Sertindol, Sulpirid, Sultopridhydrochlorid, Suriclon, Tandospironcitrat, Temazepam, Tetrabenazin, Tetrazepam, Thioproperazinmesylat, Thioridazin, Thiothixen (+Hydrochlorid), Thiapridhydrochlorid, Timiperon, Tofisopam, Triazolam, Triclofos-Natrium, Trifluoperazinhydrochlorid, Trifluperidol (+Hydrochlorid), Valnoctamid, Veraliprid, Vinylbiton, Zaieplon, Ziprasidon, Zolazepamhydrochlorid, Zolpidemtartrat, Zopiclon, Zotepin, Zuclopenthixol (+Acetat, Dekanoat, Hydrochlorid), Atropin (+Methobromid, Methonitrat und Sulfat), Belladonna, Benzhexolhydrochlorid, Benztropinmesylat, Biperiden, Bornaprinhydrochlorid, Butropiumbromid, Buzepidemetiodure, Cimetropiumbromid, Clidiniumbromid, Ciclodrinhydrochlorid, Cyclopentolathydrochlorid, Darifenacin, Desetimidhydrochlorid, Diclominhydrochlorid, Diethazinhydrochlorid, Difemerinhydrochlorid, Dihexiverinhydrochlorid, Dimevamid, Diphemanilmethylsulfat, Drofeninhydrochlorid, Emeproniumbromid (+Carragenat), Ethopropazinhydrochlorid, Eucatropinhydrochlorid, Fentoniumbromid, Flavoxathydrochlorid, Flutropiumbromid, Glycopyrroniumbromid, Homatropin (+Hydrobromid und Methobromid), Hioscin (+Butylbromid, Hydrobromid, Methobromid und Methonitrat), Hiosciamin, Hyosciamus, Isopropamid-Jodid, Mecloxamincitrat, Mepenzolatbromid, Methantheliniumbromid, Metixenhydrochlorid, Metilbenactiziumbromid, Octatropinmethylbromid, Ophenadrincitrat (+Hydrochlorid), Oxybutininhydrochlorid, Oxyphencycliminhydrochlorid, Oxyphenoniumbromid, Penthienatbromid, Fenamazidhydrochlorid, Pipenzolatbromid, Piperidolathydrochlorid, Pipethanatethobromid, Pirenzepinhydrochlorid, Poldinmethylsulfat, Pirifiniumbromid, Procyclidinhydrochlorid, Propantelinbromid, Propiverinhydrochlorid, Stramonium, Telenzepin, Terodilinhydrochlorid, Tiemonium-Jodid, Timepidiumbromid, Tolterodintartrat, Tridihexethylchlorid, Tropatepinhydrochlorid, Tropicamid, Tropinbenzylathydrochlorid, Valethamatbromid, Xenytropiumbromid, Zamifenacin, Amesergid, Amineptinhydrochlorid, Amitriptilin, Amoxapin, Befloxaton, Benactizinhydrochlorid, Brofaromin, Buproprionhydrochlorid, Butriptylinhydrochlorid, Citalopramhydrobromid, Clomipraminhydrochlorid, Clorgilinhydrochlorid, Demexiptilinhydrochloride, Desipraminhydrochlorid, Dibenzepinhydrochlorid, Dothiepinhydrochlorid, Doxepinhydrochlorid, Duloxetinhydrochlorid, Etoperidonhydrochlorid, Femoxetin, Fluoxetinhydrochlorid, Fluvoxaminmaleat, Imipramin, Iprindolhydrochlorid, Iproniazidphosphat, Isocarboxazid, Lithiumcarbonat (+Citrat), lofepraminhydrochlorid, Maprotilinhydrochlorid, Medifoxamin, Melitracenhydrochlorid, Metapraminfumarat, Mianserinhydrochlorid, Milnacipran, Minaprinhydrochlorid, Mirtazapin, Moclobemid, Nefazodonhydrochlorid, Nialamid, Nomifenisinmaleat, Nortriptylinhydrochlorid, Opipramolhydrochlorid, Oxaflozanhydrochlorid, Oxaprotilinhydrochlorid, Oxytriptan, Paroxetinhydrochlorid, Phenelzinsulfat, Pirlindol, Pivagabin, Protriptilinhydrochlorid, Quinupramin, Reboxetin, Rubidiumchlorid, Sertralinhydrochlorid, Tianeptin-Natrium, Tranylcyprominsulfat, Trazodonhydrochlorid, Trimipramin (+Maleat), Tryptophan, Venlafaxinhydrochlorid, Viloxazinhydrochlorid, Viqualin, Zimeldinhydrochlorid, Alphadolonacetat, Alphaxalon, Eltanolon, Etomidat, Ketaminhydrochlorid, Methohexiton, Propanidid, Propofol, Natriumoxybat, Thiamilal-Natrium, Tiopenthon-Natrium und Tiletamin-Hydrochlorid.

5. Pharmazeutische Formulierung gemäß Anspruch 2, wobei der wasserlösliche Wirkstoff mit anabolischer oder ähnlicher Aktivität ausgewählt wird aus der Gruppe bestehend aus Substanzen, welche dazu geeignet sind, die Leistungsfähigkeit und/oder die Muskelmasse von Sportlern zu erhöhen.

6. Pharmazeutische Formulierung gemäß Anspruch 5, wobei der Wirkstoff mit anabolischer oder ähnlicher Aktivität ausgewählt wird aus der Gruppe bestehend aus Testosteron, Clostebolacetat, Drostanolonpropionat, Estrapronicat, Ethylestrenol, Fluoximesteron, Formebolon, Mepitiostan, Mesterolon, Metandienon, Metenolonacetat, Methyltestosteron, Nandrolon (+Cyclohexylpropionat, Dekanoat, Laurat, Phenylpropionat, Natriumsulfat und Undekanoat), Norethandrolon, Oxaboloncipionat, Oxandrolon, Oxymetholon, Stanozolol und Trenbolonacetat.

7. Pharmazeutische Formulierung gemäß einem der vorherigen Ansprüche, wobei die organoleptischen Marker ausgewählt werden aus der Gruppe bestehend aus Knoblauchextrakt (Allium sativum [Liliaceae]) oder dessen Bestandteilen, z.B. Alliin, Allicin, Diallylsulfid oder Ajoen, Aloe vera-Extrakt (Aloe barbadensis, Aloe vera, Aloe capensis, Aloe ferox [Liliaceae]) oder einer oder mehreren Bestandteilen davon, Chininsulfat/hydrochlorid/bromohydrat (Cinchona [Rubiaceae]), oder Chinarindenextrakt als solchem, natürliche Farbstoffe, z.B. E100, E101, E140, E160a, E160b, E160c, E160d, E160e, E160f, E161, E161a, E161b, E161C, E161d, E161e, E161f, E161g, E162, E163, Safran und verschiedene andere Farbstoffe, z.B. E104, E110, E120, E122 E123, E124, E127, E131, E132, E141, E142, E150, E151 und E153.

## Revendications

1. Formulation pharmaceutique pour administration orale ou liposoluble sous la forme d'une capsule molle renfermant un principe actif soluble dans l'eau susceptible de rendre illicite l'administration et au moins un marqueur organoleptique pharmaceutiquement acceptable indépendamment sélectionné parmi une ou plusieurs substances appartenant au groupe consistant en un agent aromatisant hydrophile, un agent aromatisant hydrophobe, un agent colorant hydrophile, un agent colorant hydrophobe, un agent colorant hydrophobe et un odorant où ladite formulation pharmaceutique contient une suspension dans l'huile ou solution comprenant le principe actif dissous ou en suspension dans l'huile, au moins un premier marqueur organoleptique soluble dans l'eau en suspension dans l'huile, au moins un second marqueur organoleptique liposoluble dissous dans l'huile.

2. Formulation pharmaceutique selon la revendication 1 où le principe actif est sélectionné dans le groupe consistant en une substance agissant sur le système nerveux central et/ou comme narcotique et une substance avec une activité anabolisante ou analogue.

3. Formulation pharmaceutique selon la revendication 2 où le principe actif agissant sur le système nerveux central et/ou en tant que narcotique est sélectionné dans le groupe consistant en sédatif, hypnotique, antipsychotique, antidépresseur, tranquillisant et antimuscarinique.

4. Formulation pharmaceutique selon la revendication 3 où l'ingrédient actif agissant sur le système nerveux central et/ou en tant que narcotique est sélectionné dans le groupe consistant en abécarnil, acamprosate calcium, acépromazine (+maléate), acéprométazine, acétophénazine maléate, acétylglycinamide-chloralum, adinazolam mésylate, allobarbitone, aldipem, alprazolam, amisulpride, amylobarbitone (+sodium), aprobarbital, azapérone, barbitone (+sodium), benpéridol, bentazépam, brallobarbital, bromazépam, bromisoval, brompéridol (+décanoate), brotizolam, chlorohydrate de buspirone, butalbital, butobarbitone, bromolactobionate de calcium, camazépam, chlorhydrate de captodiame, carbromal, chlorhydrate de carpipramine, chloral bétaine, chloralum, chloralose, chlordiazépoxyde (+chlorhydrate), chlorhexadol, chlorméthiazole (+édisylate), chlormézanone, chlorhydrate de chloproéthazine, chlorpromazine (+embonate et chlorhydrate), chlorprotixène (+chlorhydrate et mésylate), cinaolazépam, clobazam, chlorhydrate de clocapramine, chlorazépine acide, (+sels de potassium), clotiapina, clothiazépam, cloxazolam, clozapine, cyamémazine, cyclobarbitone (+calcium), délorazépam, chlorhydrate de détomidine, desmédétomidine, diazépam, dichloralphénazone, difébarbamate, dropéridole, eltoprazine, chlorhydrate d'enciprazine, estazolam, etclorvinol, loflazépate d'éthyle, chlorhydrate d'étifoxine, étizolam, etodroxizine, fébarbamate, fluanisone, fludiazépam, flunitrazépam, flupentixol décanaote (+chlorhydrate), fluphénazine décanoate (+énanthate et chlorhydrate) flurazépam, (+monochlorhydrate et dichlorhydrate), fluspirilène, chlorhydrate de gépirone, glutéthimide, halazépam, halopéridol, haloxazolam, hexapropymate, hexobarbitone (+sodium), chlorhydrate d'homofénazine, ibomal, chlorhydrate d'ipsapirone, cétazolam, mésylate de loprazolam, lorazépam, lormétazépam, loxapine, aspartate de magnésium bromhydraté, médazépam, chlorhydrate de médétomidine, chlorhydrate de melpérone, méphénoxalone, méprobamate, mésordazine bésylate, chlorhydrate de métaclazépam, méthaqualone, méthotriméprazine, méthylpentynol, méxazolam, midazolam (+maléate), chlorhydrate de molindone, chlorhydrate de mopérone, mosapramine, némonapride, nimétazépam, nitrazépam, nordazépam, olanzapine, oxazépam, ossazolam, oxypertine, paraldéhyde, penfluridol, pentobarbitone (+calcium et sodium), dimalonate de pérazine, périciazine, perphénaziire (+décanoate et énanthate), phenprobamate, pimozide, pinazépam, chlorhydrate de pipampérone, pipothiazine, prazépam, prochlorpérazine (+édisylate, maléate et mésylate), bromure de prolonium, embonate de promazine, chlorhydrate de prothipendyle, proxibarbal, pyrithyldione, quazépame, fumarate de quetiapine, quinalbarbitone (+sodium), raclopride, chlorhydrate de rémoxipride, rispéridone, ritansérine, romifidine, secbutobarbitone (+sodium), sertindole, sulpiride, chlorhydrate de sultopride, suriclone, citrate de tandospirone, témazépame, tétrabénazine, tétrazépam, thiopropérazine mésylate, thioridazine, thiothixène (+chlorhydrate), chlorhydrate de thiapride, timipérone, tofisopam, triazolam, ticlofos sodium, chlorhydrate de trifluopérazine, triflupéridol (+chlorhydrate), valnoctamide, véralipride, vinylbitone, zalepton, ziprasidone, chlorhydrate de zolazépam, tartrate de zolpidem, zopiclone, zotépine, zuclopenthixol (+acétate, décanoate, chlorhydrate), atropine (+méthobromure, méthonitrate et sulfate), belladone, chlorhydrate de benzhexol, mésulate de benztropine, bipériden, chlorhydrate de bomaprine, bromure de butropium, métiodure de buzépide, bromure de cimétropium bromure de clinidium, chlorhydrate de ciclodrine, chlorhydrate de cyclopentolate, darifénacine, chlorhydrate de désétimide, chlorhydrate de diclomine, chlorhydrate de diéthazine, chlorhydrate de difémérine, chlorhydrate de dihexivérine, dimévamide, diphémanil méthyl sulfate, chlorhydrate de drofénine, bromure d'émépronium (+carragénate), chlorhydrate d'éthopropazine, chlorhydrate d'eucatropine, bromure de fentonium, chlorhydrate de flavoxate, bromure de flutropium, bromure de glycopyrronium, homatropine (+bromhydrate et méthobromure), hioscine (+bromure de butyle, bromhydrate, méthobromure et méthonitrate), hiosciamine, hyosciamus, iodure d'isopropamide, citrate de mécloxamine, bromure de mépenzoate, bromure de méthanthélinium, chlorhydrate de métixène, bromure de métilbenactizium, méthylbromure d'octatropine, citrate d'orphénadrine (+chlorhydrate), chlorhydrate d'oxybutinine, chlorhydrate d'oxyphencyclimine, bromure d'oxyphénonium, bromure de penthiénate, chlorhydrate de fénamazide, bromure de pipenzolate, chlorhydrate de pipéridolate, éthobromure de pipéthanate, chlorhydrate de pirenzépine, méthyl sulfate de poldine, bromure de pirifinium, chlorhydrate de procyclidine, bromure de propantéline, chlorhydrate de propivérine, stramonium, télenzépine, chlorhydrate de térodiline, iodure de tiémonium, bromure de timépidium, tartrate de toltérodine, chlorure de tridihexéthyle, chlorhydrate de tropatépine, tropicamide, chlorhydrate benzylate de tropine, bromure de valéthamate, bromure de xénytropium, zamifénacine, amésergide, chlorhydrate d'amineptine, amitriptiline, amoxapine, béfloxatone, chlorhydrate de bénactizine, brofaromine, chlorhydrate de bupropion, chlorhydrate de butyltriptyline, bromhydrate de citalopram, chlorhydrate de clomipramine, chlorhydrate de clorgiline, chlorhydrate de déméxiptiline, chlorhydrate de désipramine, chlorhydrate de dibenzépine, chlorhydrate de dothiépine, chlorhydrate de doxépine, chlorhydrate de duloxétine, chlorhydrate d'étopéridone, fémoxétine, chlorhydrate de fluoxétine, maléate de fluvoxamine, imipramine, chlorhydrate d'iprindole, phosphate d'iproniazide, isocarboxazide, carbonate de lithium (+citrate), chlorhydrate de lofépramine, chlorhydrate de maprotiline, médifoxamine, chlorhydrate de mélitracène, fumarate de métapramine, chlorhydrate de miansérine, milnacipran, chlorhydrate de minaprine, mirtazapine, moclobémide, chlorhydrate de néfazodone, nialamide, maléate de nomifenisine, chlorhydrate de nortriptyline, chlorhydrate d'opipramol, chlorhydrate d'oxaflozan, chlorhydrate d'oxaprotiline, oxytriptan, chlorhydrate de paroxétine, sulfate de phénelzine, pirlindole, pivagabine, chlorhydrate de protriptiline, quinupramine, réboxétine, chlorure de rubidium, chlorhydrate de sertraline, tianeptine sodium, sulfate de tranylcypromine, chlorhydrate de trazodone, trimipramine (+maléate), tryptophane, chlorhydrate de venlafaxine, chlorhydrate de viloxazine, viqualine, chlorhydrate de zimeldine, acétate d'alphadolone, alphaxalone, eltanolone, étomidate, chlorhydrate de cétamine, méthohexitone, propanidid, propofol, oxybate de sodium, thiamilal sodium, tiopenthone sodium, chlorhydrate de tilétamine.

5. Formulation pharmaceutique selon la revendication 2, où le principe actif soluble dans l'eau ayant une activité anabolisante ou analogue est sélectionné dans le groupe consistant en substances appropriées pour améliorer les performances des sportifs et/ou augmenter la masse musculaire.

6. Formulation pharmaceutique selon la revendication 5 où le principe actif ayant une activité anabolisante ou analogue est sélectionné dans le groupe consistant en testostérone, acétate de clostébole, propionate de drostanolone, estrapronicate, éthylestrénol, fluoximestérone, formébolone, mépitiostan, mestérolone, métandiénone, acétate de méténolone, méthyltestostérone, nandrolone (+cyclohexylpropionate, décanoate, laurate, phénylpropionate, sulfate de sodium et undécanoate), noréthandrolone, cipionate d'oxabolone, oxandrolone, oxymétholone, stanozolol et acétate de trenbolone.

7. Formulation pharmaceutique selon l'une quelconque des revendications précédentes, où les marqueurs organoleptiques sont sélectionnés dans le groupe consistant en extrait d'ail (allium sativum [Liliaceae]) ou leurs constituants, e.g. alliine, allicine, diallylsulfure ou ajoène, d'extrait d'aloe vera (aloe barbadensis, aloe vera, aloe capensis, aloe ferox [Liliaceae]) ou un ou plusieurs composants, de sulfate/chlorhydrate/bromhydrate de quinine (Cinchona [Rubiaceae]), ou d'extrait de quinine tel quel, d'agents colorants naturels, e.g. E100, E101, E140, E160a, E160b, 160c, E160d, E160e, E160f, E161, E161a, E161b, E161c, E161d, E161e, E161f, E161g, E162, E163, safran, d'agents colorants divers, e.g. E104, E110, E120, E122, E123, E124, E127, E131, E132, E141, E142, E150, E151 et E153.
